# EUROPEAN PATENT APPLICATION

(11) **EP 4 316 506 A1**
(43) Date of publication of application: **07.02.2024**
(21) Application number: 22723703.9
(22) Date of filing: 25.03.2022
(51) Int. Cl.: A61K 38/38, A61P 37/00

(54) **USE OF ALBUMIN FOR THE TREATMENT OF DEFECTIVE B-CELL FUNCTION**

(30) Priority: 25.03.2021 EP 21382248; 27.05.2021 EP 21382481
(71) Applicant: Grifols Worldwide Operations Limited, Dublin 22 (IE); European Foundation for the Study of Chronic Liver Failure (EF-CLIF), 08021 Barcelona (ES)
(72) Inventor: ARROYO PEREZ, Vicente, 08021 BARCELONA (ES); MOREAU, Richard, 95880 ENGHIEN LES BAINS (FR); CLARIA ENRICH, Joan, 08635 SANT ESTEVE SESROVIRES (BARCELONA) (ES); FERNANDEZ GOMEZ, Javier, 17002 GIRONA (ES); NUÑEZ DOMENECH, Laura, 08174 SANT CUGAT DEL VALLES (BARCELONA) (ES); HORRILLO SAURA, Raquel, 08150 PARETS DEL VALLES (BARCELONA) (ES)
(74) Representative: HGF
(86) International application number: PCT/ES2022/070171
(87) International publication number: WO 2022/200663

(57) **Abstract**

The present invention refers to a composition comprising human albumin for regulating B-cell function in a patient suffering from systemic inflammatory response syndrome, in which the albumin is administered to the patient in a dose sufficient to increase B-cell Receptor density in a B-cell population.

## Description

The present invention relates to the medical field, in particular to the use of albumin for the treatment of defective B-cell function in a patient in need thereof.

The immune system is a conglomerate of cells and molecules that cooperate to protect human beings from infectious agents. The immune system also provides human beings with a surveillance mechanism to continuously monitor the integrity of cells or tissues. By this surveillance, it is recognized changes in major histocompatibility complex proteins or viral proteins on the cell membrane that signals cancer development or intracellular viral infection. The immune system functions under two major principles: (1) recognition of foreign (non-self) material by highly sensitive plasma membrane or intracellular receptors present in the immune cells, which results in overexpression of hundreds of genes regulating the inflammatory response and proliferation of the immune cells; (2) elimination of microorganisms by a diverse repertoire of mechanisms, including unspecific anti- microbial proteins synthesized by the liver (i.e. the complement system which kills bacteria either by direct lysis or activating phagocytosis by the immune cells); the release by granulocytes, T-cytotoxic lymphocytes (Tc cells) and natural killer (NK) cells of intracellular cytotoxic granules that contains a battery of enzymes and pore-forming proteins (perforins) capable to kill microorganisms, phagocytosis (granulocytes and macrophages), and specific antibodies [B-lymphocytes (B-cells)]. Activated Tc and NK cells present Fas-ligands that interact with Fas-receptors on the target cells (cancer or virus infected cells) leading to cell-death by apoptosis. Antibodies are complex proteins synthesized by B-cells with a hypervariable region for antigen recognition and constant regions for activating the complement pathway and binding phagocytes and NK-cells for cellular lysis (i.e., antibody-dependent cell cytotoxicity) and phagocytosis (i.e., antibody mediated cellular phagocytosis). Antibodies, complement, and granulocytes give protection against most extracellular organisms, whereas macrophages, Tc-cells and NK cells are involved in the surveillance process and elimination of tumoral cells and virally infected cells. Each immune system works complementarily.

The innate immune system serves as a rapid reaction force that deploys within minutes a range of non-specific (but highly effective) weapons to eradicate the infectious agent or to keep the infection contained. Cells from the innate immune system use conserved non-specific pattern-recognition receptors (PRRs) that very reliably recognize common pathogen-associated molecular pattern (PAMPs) or damage associated molecular patterns (DAMPs) released during tissue damage. The recognition of PAMPs and DAMPs and the secondary immune response is a simple process and occurs within minutes. The innate immune system is composed by granulocytes (neutrophils, basophils, and eosinophils), macrophages (tissue cells derived for the circulating monocytes) and dendritic cells (specialized cells in presenting antigens to T, B and NK cells for the immune response). The NK cells, although being lymphoid cells, are also part of the innate immune system.

The adaptive (or acquired) immune system is a more complex and sophisticated force that deploys also very effective but extremely specific weapons. It comprises T and B lymphocytes, which can generate new specific receptors: T-cell receptor (TCR) and B-cell receptor (BCR) for every new antigen in response to each new pathogen that enters in the body, and exhibit immunological memory, whereby they respond more rapidly and vigorously upon re-encounter with specific antigen. Each B-cell is programmed to make only one specificity of antibody and it places a transmembrane version of these antibodies on its cell surface to act as receptor (B-cell receptor) of the specific antigen. The B-cells give rise to plasma cells, which produce large amounts of soluble antibodies that are secreted into the environment and systemic circulation or be transported to mucosal surfaces. In contrast with the innate system, the activation of the adaptive-immune system, i.e. antibody response, may take 10 days for a new infection and 5 days for a repeated infection.

When a microorganism re-enters in the body, it will only bind to those lymphocytes that possess receptors specifically designed for it. This phenomenon, called clonal selection, is the mechanism of the immunological memory, and leads to activation and proliferation of the original clone of plasma cells to make specific antibodies. T-cell lymphocytes with a TCR of appropriated specificity are similarly selected. T-cells can be divided into 3 broad subsets: helper (CD4 or Th), cytotoxic (subset of CD8 T-cells called Tc) and regulatory (Treg) whose respective role is helping B-cells to make antibodies (Th), killing virally infected and cancer cells (Tc) and controlling the actions of other T-cells (Treg).

As indicated, B-cell receptors (BCR) are specific immunoglobulins (IgM and IgD) anchored on the B-cell plasma membranes and connected to a complex intracellular mechanism responsible for the activation of many genes that promote the synthesis and release of specific antibodies. Although BCR can be stimulated by soluble antigens, the primary form that triggers B-cell activation is through follicular dendritic cells that are resident within the lymph nodes and spleen and present antigens to T, B and NK cells. Activation of B-cells is a complex process which relies not only on the interaction of BCRs with antigens but also on co-stimulation mechanisms. One form of co-stimulation takes place at the same time of the initial encounter of the BCR with the specific antigen and is provided by the B-cell co-receptor complex in the cell membrane. The other form of co-stimulation is provided by activated Th-cells, in the form of membrane associated CD-40 ligands that engages with the surface CD-40 on B-cell, and cytokines (T-cell-dependent co-stimulation). B-cell, however, may present also PRRs (such as Toll-like receptors), and function as innate immune cell giving rise to a polyclonal T-cell-independent rapid response to PAMPs or DAMPs and release of polyclonal antibodies. In this way, B-cells collaborate in the rapid alarming reaction against infection. B-cells may also function as antigen-presenting cell, and, together with dendritic cells and macrophages, participate in the activation of T-cells.

There are three types of B-cells that respond to infections by secreting antibodies that target specific classes of microbes/antigens, with the particular function of each determined by their location and the type of antigen they react with. The follicular B-cells (also called B2 cells) express highly specific monoreactive BCRs, are present in the lymphoid follicles of the lymph nodes and spleen, and typically target thymus-dependent antigens requiring Th-cells to generate appropriated monoclonal antibody responses. However, certain antigens (Type 1 and type-2 thymus-independent antigens), such as bacterial lipopolysaccharides, when present at high concentrations, or lineal antigens that are not readily degraded in the body, have the ability to activate a substantial proportion of B-cells polyclonally without the help of T-cells giving rise to T-independent polyclonal antibody response. They do this through binding to PRR (i.e. Toll-like receptors) or polyreactive BCRs on the surface of B-cells. This T-cell independent detection of microbial antigen is performed by two types of extrafollicular B-cells: the marginal zone (MZ) B-cells and the B1 cells.

The association of systemic inflammation, immunosuppression, high prevalence of secondary bacterial infections and multiorgan system failure identifies a syndrome commonly observed in critically ill patients, including patients with acutely decompensated cirrhosis, acute liver failure, severe sepsis (including septic shock), acute pancreatitis and other intraabdominal inflammatory processes, polytrauma, burns or Covid 19. Among patients with acutely decompensated cirrhosis, immune activation is more marked in those with acute-on-chronic liver failure (ACLF), a syndrome characterized by intense systemic inflammation, multiorgan dysfunction/failure and extremely high prevalence of bacterial infections (Moreau et al., 2013, Gastroenterology,144:1426-37, 1437.e1-9; Arroyo, et al., 2020, N Engl J Med, 382:2137-2145) than among those without ACLF (Weiss et al., 2021, Frontiers in Immunol, in press). Moreover, the PREDICT study has recently identified a subset of patients with severe systemic inflammation but without ACLF who are at high risk of developing ACLF and to die during hospitalization or within few weeks after discharge (Trebicka et al., 2020, J Hepatol, 73:842-854). This subset of patients is classified as pre-ACLF patients. Finally, although there is no established therapy against immunosuppression to date, recent studies by the inventors suggests that intravenous albumin, beyond its plasma expander properties, may reduce the severity of systemic inflammation (Fernández et al., 2018, Gut, 67:1870-1880; Casulleras et al., 2020, Sci Transl Med, 12(566):eaax5135).

Tumors may generate different types of antigens, promote activation of the immune system and lead to inflammatory response. Primary tumors and their metastasis are infiltrated by innate and adaptive immune cells. Additionally, tumors may develop tertiary lymphoid structures (TLSs), which function as ectopic lymphoid organs in response to tumoral antigens (Sautès-Fridman et al., 2019, Nat Rev Cancer, 19:307-325; Helmink et al., 2020, Nature, 557:549-555). TLSs exists under different maturation states, culminating in the formation of germinal centers, which are sites where mature B-cells proliferate, differentiate, and where antibody-V region somatic hypermutation and selection events occur. TLSs are thought to actively modulate anti-tumor adaptive immunity. Although T lymphocytes and NK cells are the main cells participating in the immune response to tumors, recent studies indicates that B-cell is also important.

Cancer deploys multiple strategies to evade and repel the immune attack. Among these strategies, those related with immune T-cell checkpoints (CTLA-4 and PD-1) are of relevance because they have determined the development of an extremely effective new approach for the treatment of melanoma and other tumors. Checkpoint molecules are upregulated on activated T-cells and play an important physiological role terminating T-cell response once it is no longer needed. In this way they prevent the occurrence of autoimmune reactions. CTLA-4 suppresses T-cell function by competing with CD28 binding on T-cell. PD-1 actively suppresses T-cell signal transduction pathway. The engagement of these T-cell checkpoints to partner proteins on tumor cells (e.g., PDL-1 engaging PD-1) leads to a permanent state of tolerance favoring tumor expansion. In contrast, treatment with checkpoint blocking antibodies restores the immune system function allowing the innate immune system to attack cancer cells (Helmink et al., 2020, Nature, 557:549-555).

Several evidences suggest that B-cells are associated with the outcome and response to treatment using checkpoint blockade and other treatments (1) B-cells, together with other immune cells, frequently infiltrate a wide range of human cancer; (2) there is correlation between clinical benefit and presence and density of B-cells and TLSs in patients treated with immune checkpoint blockade; (3) B-cell likely drives the humoral antitumor response within TLS; (4) higher expression of B-cell related genes in tumor samples is observed in patients with cancer that respond to immune checkpoint blockade (ICB) versus not responding patients, with over-representation of these genes compared to T-cells and other immune markers. B-cells also have been shown to be positively associated with response to chemotherapy in other cancer types; (5) architectural analysis showed that B-cells were localized in mature TLSs of tumor cell responders and colocalized with Th and Tc lymphocytes; (6) tumors from ICB responders have a significantly higher frequency memory B-cells, whereas non-responders have a significantly higher frequency of naive B-cells. Other notable differences include an increased in plasma cells, switched memory B-cells and germinal center-like B-cells in responders compared with non-responders. These data are consistent with a role of B-cells in treatment response to cancer.

The immune checkpoint blockade (ICB) is currently approved by the Food and Drug Administration (FDA) for the treatment of Hodgkin lymphoma and breast, cervical, bladder, colon, head and neck, liver, lung, renal cell, skin, stomach and rectal cancer.

The inventors have surprisingly found that albumin selectively activates the B-cell function without affecting the T-cell compartment. By doing this, albumin rescues B-cells from the generalized under- regulated dysfunction of the adaptive immune cells.

In a first aspect, the present invention refers to a composition comprising human albumin for regulating B-cell function in a patient suffering from systemic inflammatory response syndrome, wherein the albumin is administered to the patient in a dose sufficient to increase B-cell Receptor density in a B-cell population selected from the group consisting of naive B-cells, transitional B-cells, marginal B-cells and combinations thereof such that the B-cell Receptor density increases by at least about 10 % compared to the pre-treatment B-cell Receptor density, as determined by flow cytometry.

Preferably, the patient suffering from systemic inflammatory response syndrome is a sepsis patient or a patient with a liver condition.

Preferably, the patient suffering from systemic inflammatory response syndrome has a liver condition selected from the group consisting of decompensated cirrhosis, pre-acute-on-chronic liver failure, and acute-on-chronic liver failure. More preferably, the liver condition is acute-on-chronic liver failure.

Preferably, the B-cell Receptors comprise an immunoglobulin selected from the group consisting of an IgD subtype, an IgM subtype, and combinations thereof.

Preferably, the B-cell Receptor density increases by at least about 20 % compared to the pre-treatment B-cell Receptor density, for example at least about 30 %, such as about 40 % compared to the pre-treatment B-cell Receptor density. In some embodiments the B-cell Receptor density may increase by at least about 50 %, for example at least about 60 %, such as at least about 70 %, or in some cases at least about 80 % compared to the pre-treatment B-cell Receptor density.

In a second aspect, the present invention refers to a composition comprising human albumin for the treatment of defective B-cell function in a patient in need thereof.

Preferably, said defective B-cell function is related to a disease selected from the group comprising systemic inflammatory response syndrome, decompensated cirrhosis, acute liver failure, acute on chronic liver failure, severe sepsis and septic shock, Coronavirus 19 Disease (Covid 19), severe polytraumatic conditions, severe burns, severe acute pancreatitis, hemophagocytic syndrome, heat-shock syndrome, acute ischemia-reperfusion syndrome, inflammation diseases, and cancer.

Preferably, said cancer is selected from the group comprising Hodgkin lymphoma, breast cancer, cervical cancer, bladder cancer, colon cancer, head and neck cancer, liver cancer, lung cancer, renal cell cancer, skin cancer, stomach cancer and rectal cancer.

Preferably, the albumin is administered by an administration route selected from the group consisting of intravenous, subcutaneous, intramuscular, intradermal, intraperitoneal, intrapulmonary, intranasal, oral, rectal, and combinations thereof.

Preferably, the albumin is administered together with other treatments such as immune checkpoint blockade agents (ICB).

Preferably, the albumin is administered at multiple intervals as part of a multiple-dosage regimen.

Preferably, the multiple dosing regimen comprises multiple portion doses administered at equally spaced intervals of from about 1 day to about 30 days; or the multiple dosing regimen comprises multiple portion doses administered at unequally spaced intervals of from about 1 day to about 30 days; or the multiple dosing regimen comprises two or more administrations up to a total cumulative dose.

Preferably, the albumin is administered at individual doses of about 5 g/interval to about 500 g/interval as part of a multiple-dosage regimen; or the albumin is administered at individual doses of about 20 g/interval to about 200 g/interval as part of a multiple-dosage regimen.

Preferably, the dosing interval is every 10 days, or less.

Preferably, the albumin is human plasma-derived albumin or recombinant albumin.

Preferably, the concentration of albumin is between 4 % and 25 % (w/v); or the concentration of albumin is 20 % (w/v).

### BRIEF DESCRIPTION OF THE FIGURES

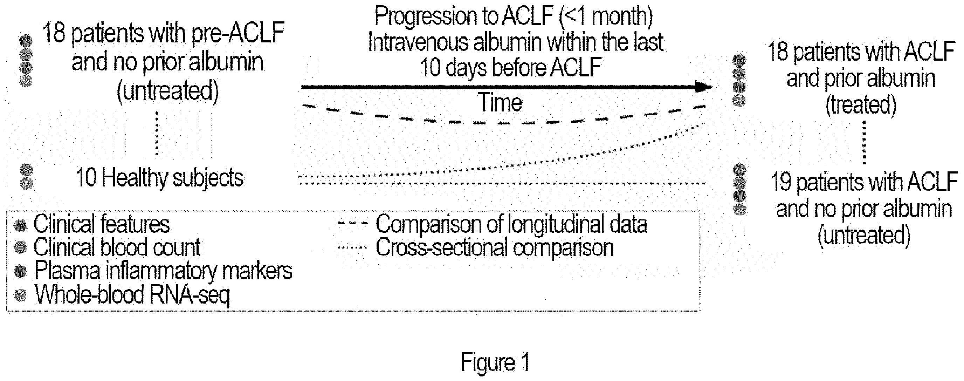

Figure 1 to 3 show an overview of the discovery Cohort 1, assessment of blood immune-cell types using gene-expression data, and effect of albumin dosage on B-cell populations.
Figure 1 shows an overview of the collection of clinical data and blood specimens in patients included in the discovery Cohort 1. Eighteen patients with acutely decompensated cirrhosis who were longitudinally studied before and after they had received intravenous albumin. These 18 patients had pre-ACLF before albumin and ACLF after albumin treatment (indicating that they were first in a pre-ACLF-untreated state and then in ACLF-treated state). The timeline between these two study points is indicated by the horizontal continuous arrow. This panel also shows data collected in 19 patients with ACLF who had not received albumin (and therefore were investigated when they were in the untreated state) and 10 healthy subjects. The hatched line indicates the comparisons that were performed using longitudinally obtained data. The dotted lines indicate the cross-sectional comparisons that were performed.

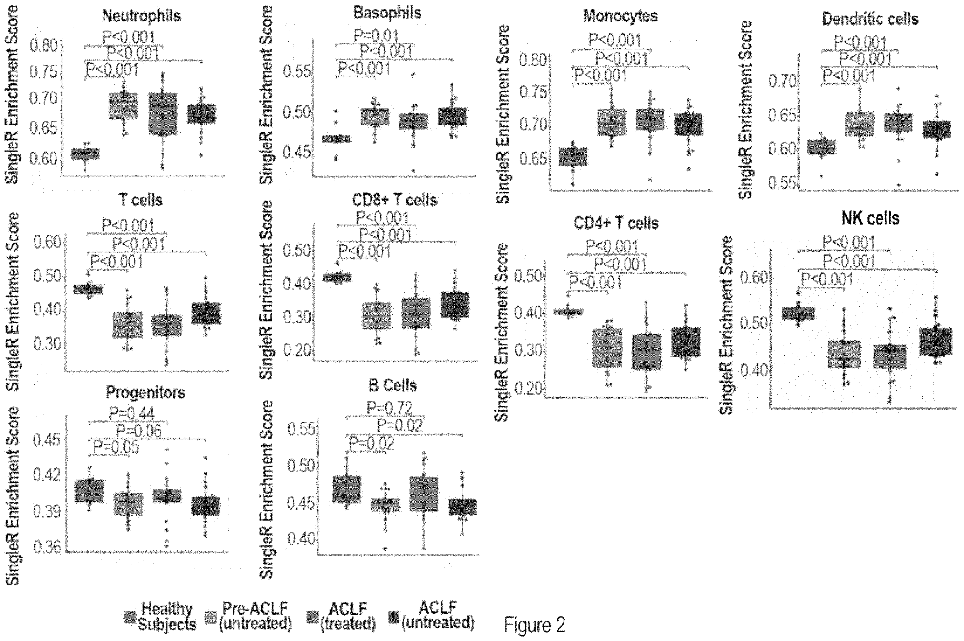

Figure 2 shows the box plots of SingleR enrichment scores for 10 immune-cell types, including neutrophils, basophils, monocytes, dendritic cells, T-cells, CD4 T-cells, CD8 T-cells, progenitors, B-cells, and NK cells computed using whole-blood RNA-seq data obtained in individuals included in the discovery Cohort 1. The horizontal line in each box represents the median, the lower and upper boundaries of the boxes the interquartile range, and the whiskers show 1.5 times the interquartile range. The P values are from Kruskal-Wallis tests followed by Mann-Whitney U tests.

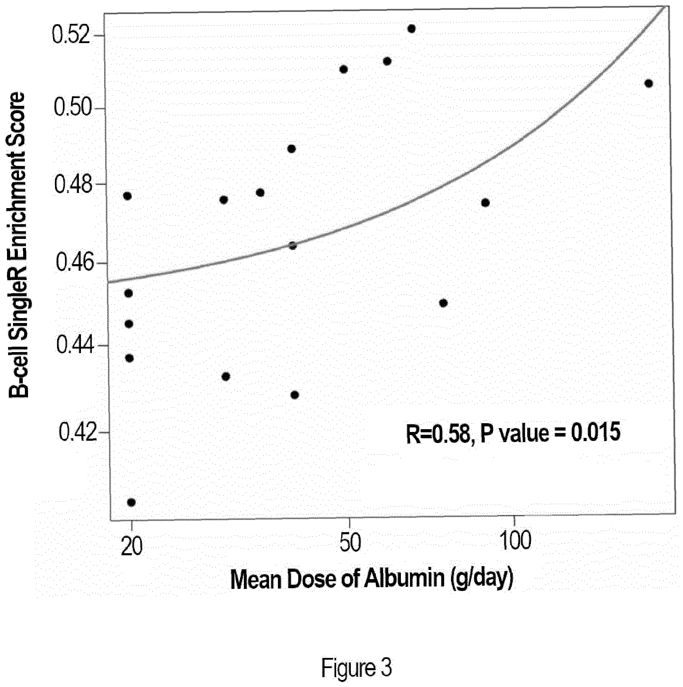

Figure 3 shows the correlation between the daily dose of albumin and the SingleR enrichment score for B-cells in patients of the discovery Cohort 1. The R value was a Spearman's correlation coefficient.

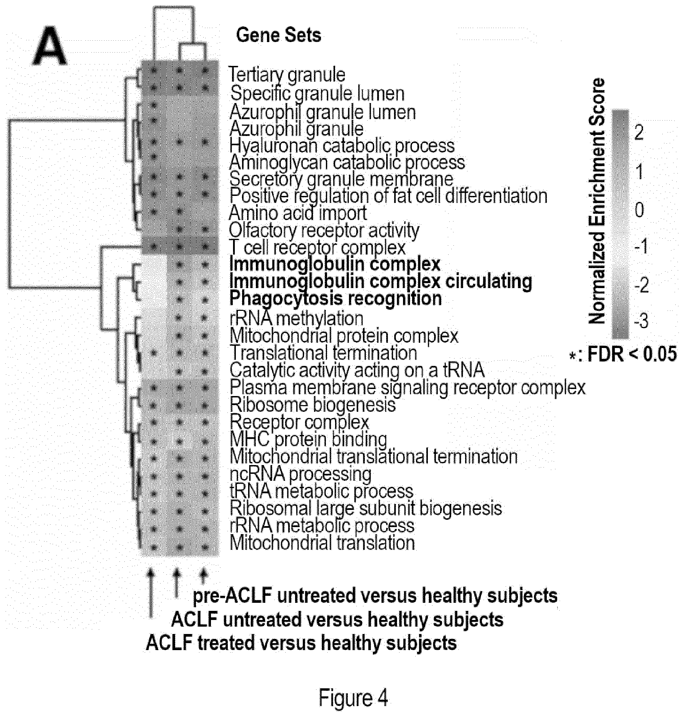

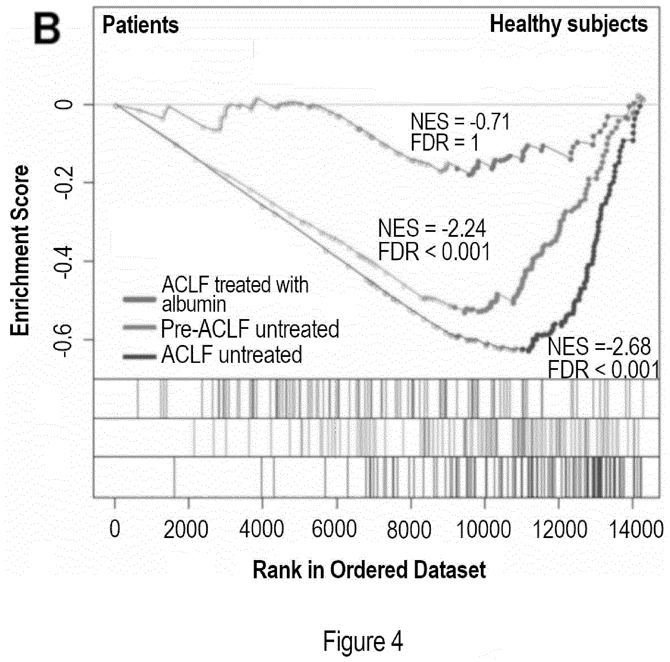

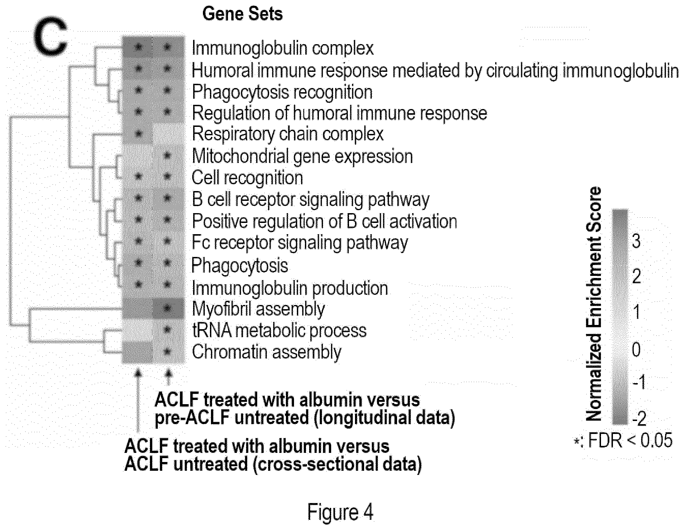

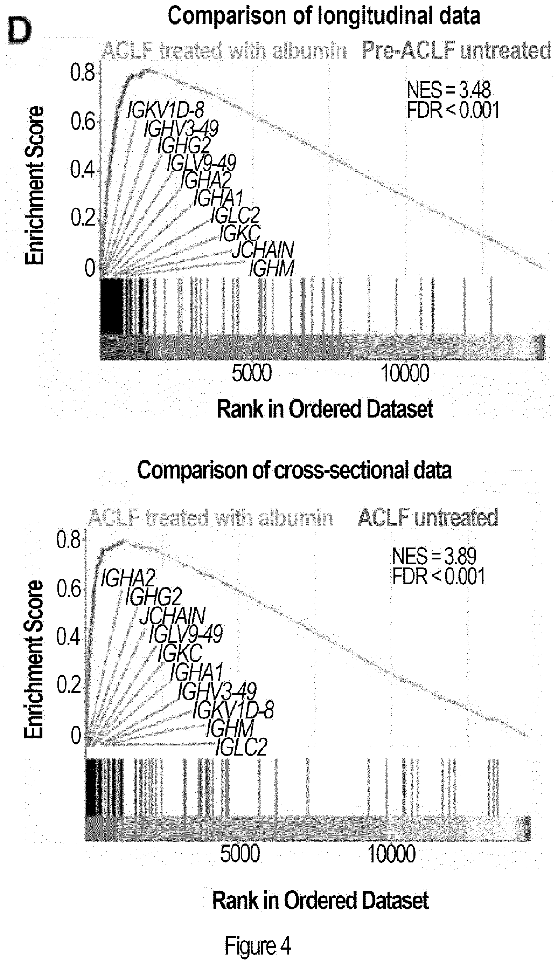

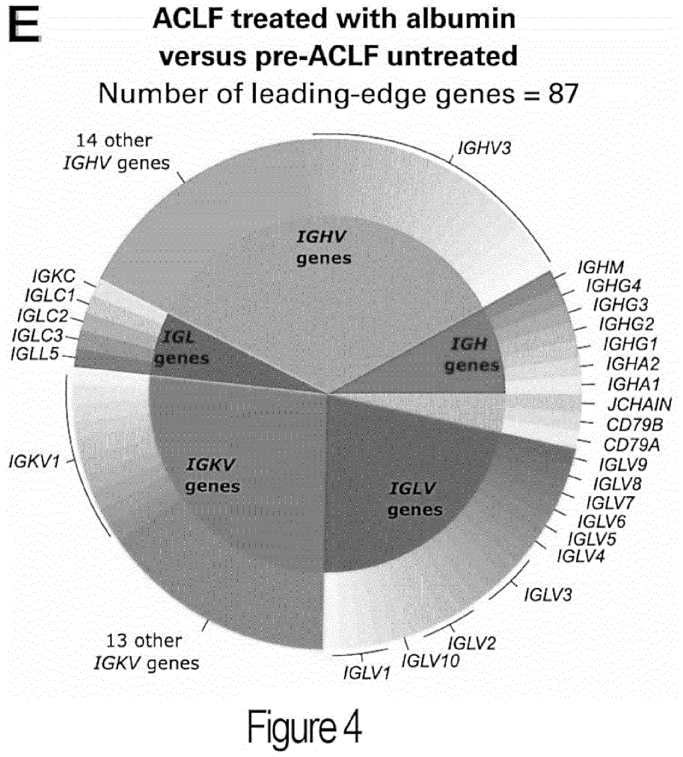

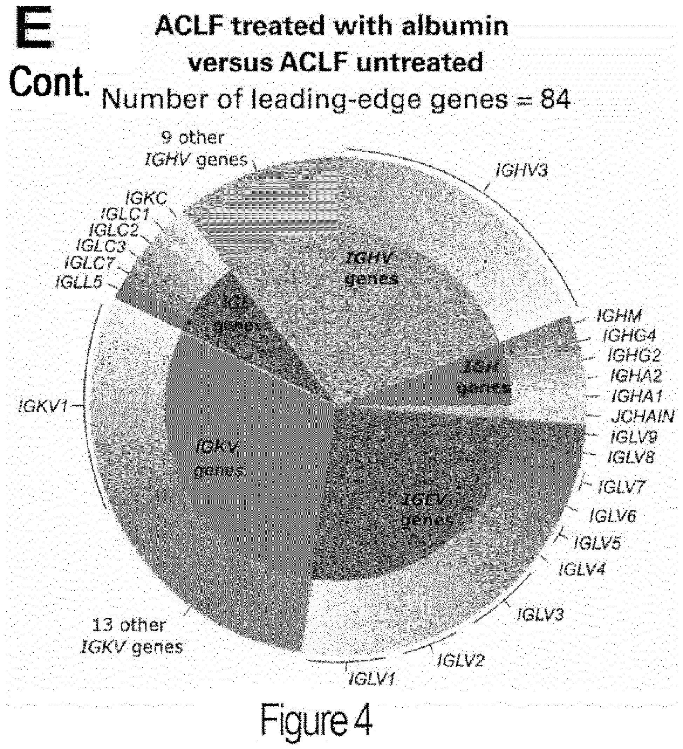

Figure 4 shows the effects of albumin on transcriptional characteristics in blood from patients with acutely decompensated cirrhosis included in the discovery Cohort 1.

Panels A through E have been designed using results of whole-blood RNA sequencing data submitted to GSEA (www.broad.mit.edu/gsea). GSEA generated a rank ordered lists of genes for each of the pairwise comparisons indicated in Fig. 1. Each rank ordered list of genes was used by GSEA for gene-set enrichment analysis using Gene Ontology gene sets. Panel A shows hierarchical cluster analysis of GSEA normalized enrichment scores (NES) of 28 gene sets across 3 cross-sectional pairwise comparisons: (1) patients who were in the pre-ACLF-untreated state versus healthy subjects, (2) patients in the ACLF-treated state versus healthy subjects, and (3) patients in the ACLF-untreated state versus healthy subjects. The 28 gene sets were identified from the analysis of the top 40 absolute values of significant NES in each comparison. Color gradient corresponds to the NES of gene sets that were downregulated (in blue) or upregulated (in red), in patients relative to healthy subjects. Gene sets indicated in bold were significantly downregulated in 2 comparisons (patients who were in the pre-ACLF-untreated state versus healthy subjects and patients in the ACLF-untreated state versus healthy subjects) but not significantly enriched in patients in the ACLF-treated state versus healthy subjects. The presence of stars in certain cells of the heat map indicates a false discovery rate (FDR) <0.05. Panel B shows GSEA enrichment plots of the immunoglobulin complex gene set in 3 cross-sectional pairwise comparisons of patients versus healthy subjects. The leading edge (i.e., top scoring) genes are indicated by solid dots. The hash plots under GSEA curves show where the members of the gene set appear in each of the 3 ranked lists of genes. Right-leaning curves (negative enrichment scores) indicate lower expression in patients. Sigmoidal curves indicate equivalent expression between the groups being compared. The NES and FDR testing the significance of each comparison are indicated. Panel C shows hierarchical cluster analysis of GSEA NES of 15 gene sets in 2 comparisons involving longitudinally collected data (ACLF-treated state versus pre-ACLF-untreated state), and cross-sectional data (ACLF-treated state versus ACLF-untreated state). The 15 gene sets were identified from the analysis of the top 40 absolute values of significant NES in each comparison. Color gradient corresponds to the NES of gene sets that were downregulated (in blue) or upregulated (in red) in treated patients relative to untreated patients. The star in certain cells of the heat map indicates significance (FOR <0.05). Panel D shows the GSEA enrichment plots of the immunoglobulin complex gene set in 2 comparisons involving longitudinally collected data (ACLF-treated state versus pre-ACLF-untreated state; top of the panel), and cross-sectional data (ACLF-treated state versus ACLF-untreated state; bottom of the panel). The hash plot under GSEA curves shows where the members of the gene set appear in the ranked list of genes. The genes shown on the plots are representative of leading edge genes that are shared by the 2 comparisons. The NES and FDR testing the significance of each comparison are indicated. Panel E show the pie plots describing the leading-edge genes in 2 comparisons involving longitudinally collected data (ACLF-treated state versus pre-ACLF-untreated state), and cross-sectional data (ACLF-treated state versus ACLF-untreated state). GSEA denotes gene set enrichment analysis.

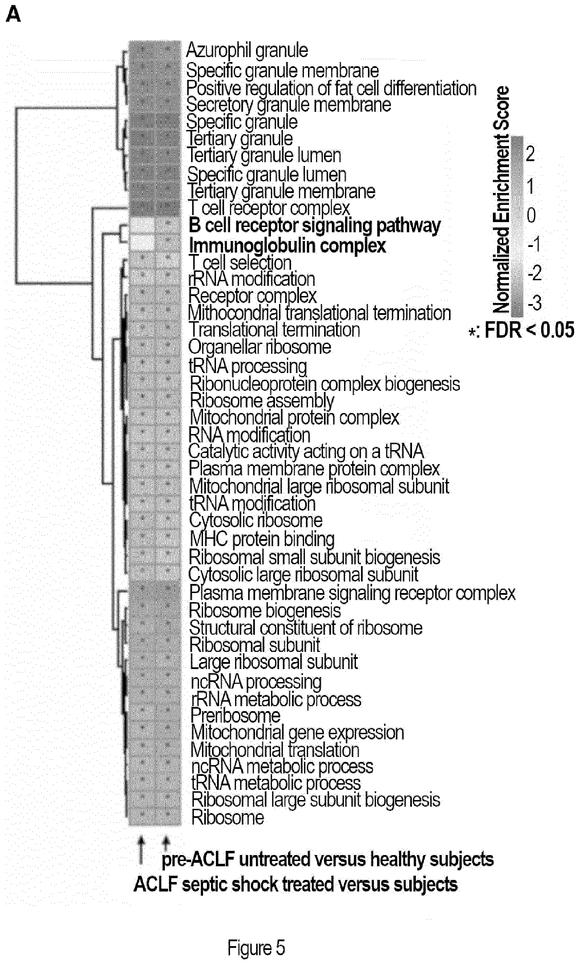

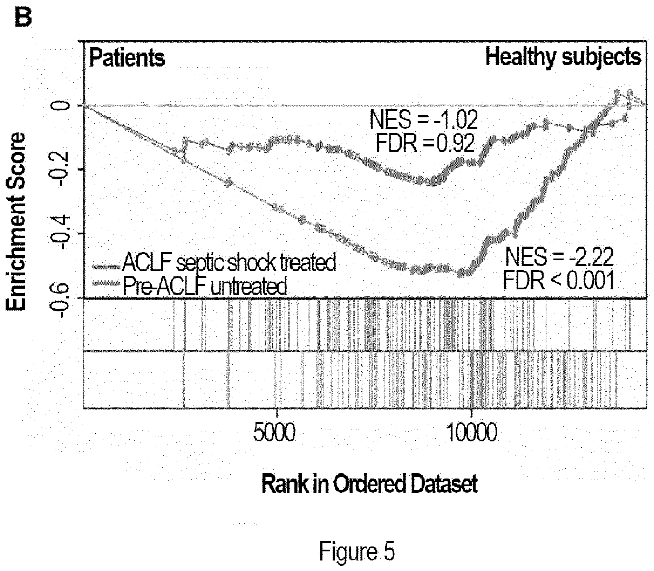

Figure 5 shows the effects of albumin on transcriptional characteristics in blood from 12 patients with acutely decompensated cirrhosis and septic shock included in the discovery Cohort 2.

Panels A and B have been designed using results of whole-blood RNA sequencing data submitted to GSEA (www.broad.mit.edu/gsea). GSEA generated a rank ordered lists of genes for each of the pairwise comparisons indicated in panel A. Each rank ordered list of genes was used by GSEA for gene-set enrichment analysis using Gene Ontology gene sets. Panel A shows hierarchical cluster analysis of GSEA normalized enrichment scores (NES) of 45 gene sets across 2 cross-sectional pairwise comparisons: (1) patients who were in the pre-ACLF-untreated state versus healthy subjects, (2) patients in the ACLF-treated state secondary to septic shock versus healthy subjects. The 45 gene sets were identified from the analysis of the top absolute values of significant NES in each comparison. Color gradient corresponds to the NES of gene sets that were downregulated (in blue) or upregulated (in red), in patients relative to healthy subjects. Gene sets indicated in bold were significantly downregulated in one comparison (patients who were in the pre-ACLF-untreated state versus healthy subjects) but not significantly enriched in patients in the ACLF-treated with septic shock state versus healthy subjects. The presence of stars in certain cells of the heat map indicates a false discovery rate (FDR) <0.05. Panel B shows GSEA enrichment plots of the immunoglobulin complex gene set in 2 cross-sectional pairwise comparisons of patients versus healthy subjects. The leading edge (i.e., top scoring) genes are indicated by solid dots. The hash plots under GSEA curves show where the members of the gene set appear in each of the 2 ranked lists of genes. Right-leaning curves (negative enrichment scores) indicate lower expression in patients. Sigmoidal curves indicate equivalent expression between the groups being compared. The NES and FDR testing the significance of each comparison are indicated.

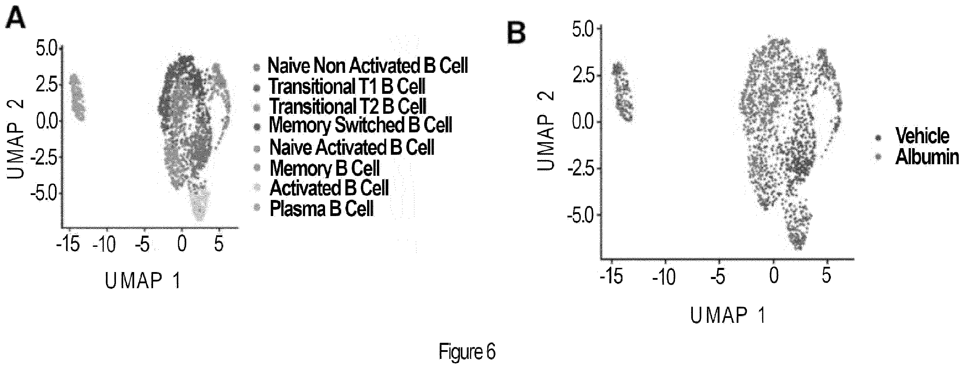

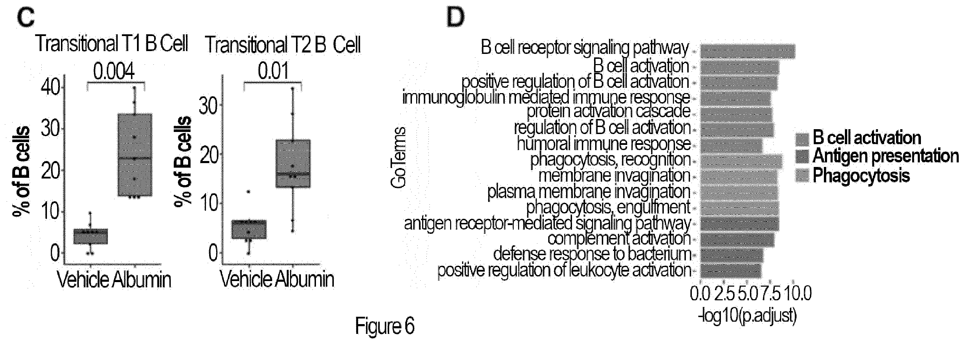

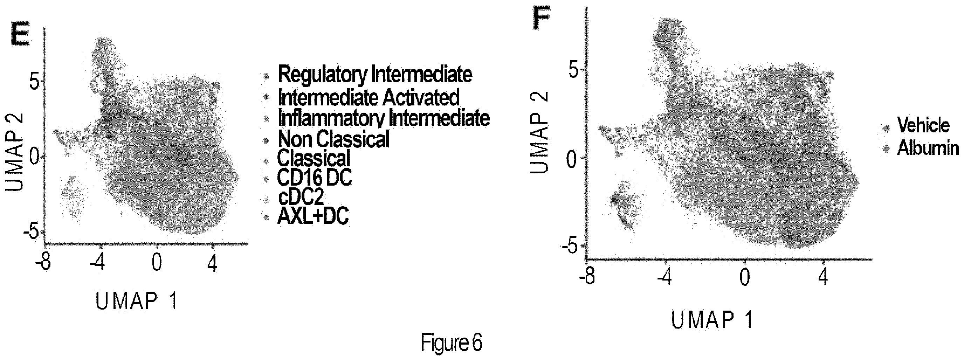

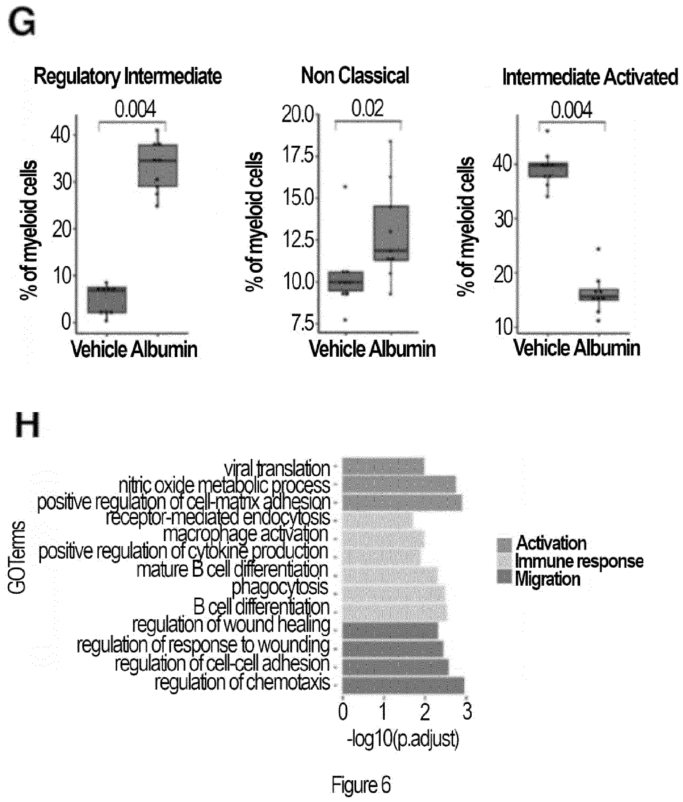

Figure 6 shows *ex vivo* effects of albumin on transcriptional characteristics of B-cell and monocyte populations from 9 patients with acutely decompensated cirrhosis included in the post-discovery Cohort 1.

Fresh peripheral blood mononuclear cells (PBMCs) were exposed *ex vivo* to albumin (15 mg/ml) or vehicle; after 2-hour exposure, PBMC single-cell RNA-sequencing (scRNA-seq) was performed.

Panel A corresponds to the uniform manifold approximation and projection (UMAP) of 1,834 cells colored by B-cell and plasma cell states. The B-cell UMAP shows naive non activated B-cells, naive activated B-cells, transitional T1 and T2 B-cells, memory B-cells, memory switched B-cells, activated B-cells and plasma B-cells. Panel B shows B-cell (on the right) and plasma B-cell (on the left) UMAP colored by *ex vivo* exposure to albumin or vehicle in B-cell and plasma cell states. Panel C shows B-cells abundance comparison among PBMCs cultured *ex vivo* with albumin or vehicle. Abundance was calculated as a fraction of total number of B-cells across each patient for transitional T1 and transitional T2 cell states comparing cells treated with vehicle or albumin with Paired Wilcoxon Signed-Rank test for patients. In Panel C, boxes show the median and interquartile range (IQR) for each group of cells treated with vehicle or albumin, with whiskers extending to 1.5× the IQR in either direction from the top or bottom quartile, each point represents a different patient with PBMCs cultured *ex vivo* with albumin or vehicle. Panel D shows a bar plot summarizing Gene Ontology over representation of transitional (T1 and T2) B-cell gene markers obtained from PBMCs cultured *ex vivo* only with albumin. Over represented Gene Ontology terms are divided in three main subcategories: B-cell activation, antigen presentation and phagocytosis. Panel E shows the UMAP of 20,396 cells colored by myeloid cell subpopulations obtained from PBMCs cultured *ex vivo* with either albumin or vehicle.

The inventors identified different cell states of monocytes: classical monocytes, inflammatory intermediate, intermediate activated, regulatory intermediate monocytes and non-classical monocytes. Furthermore, dendritic cell (DC) compartment is composed by conventional DC cells (cDC), AXL+ DC cells and CD16+ DC cells. Panel F shows the myeloid cells UMAP colored by *ex vivo* exposure to albumin or vehicle on monocytes and dendritic cell subpopulations among PBMCs. Panel G shows box plots with abundance comparison among PBMCs cultured *ex vivo* with albumin or vehicle. Abundance was calculated as a fraction of total number myeloid cells across each patient for regulatory intermediate monocytes, non-classical monocytes, inflammatory intermediate and intermediate activated monocytes among PBMCs that were cultured *ex vivo* with albumin or vehicle. All comparison of percentages were tested for significance by Paired Wilcoxon Signed-Rank test. Panel H shows a bar plot summarizing Gene Ontology over representation of regulatory intermediate monocytes gene markers obtained from PBMCs cultured *ex vivo* only with albumin. P value cut-off on enrichment test<0.05, p-adjusted method Benjamini-Hochberg and minimal size of genes annotated by ontology testing of 10. Gene ontology gene sets enriched among regulatory intermediate monocytes gene markers are divided in three main subcategories: activation, immune response and migration.

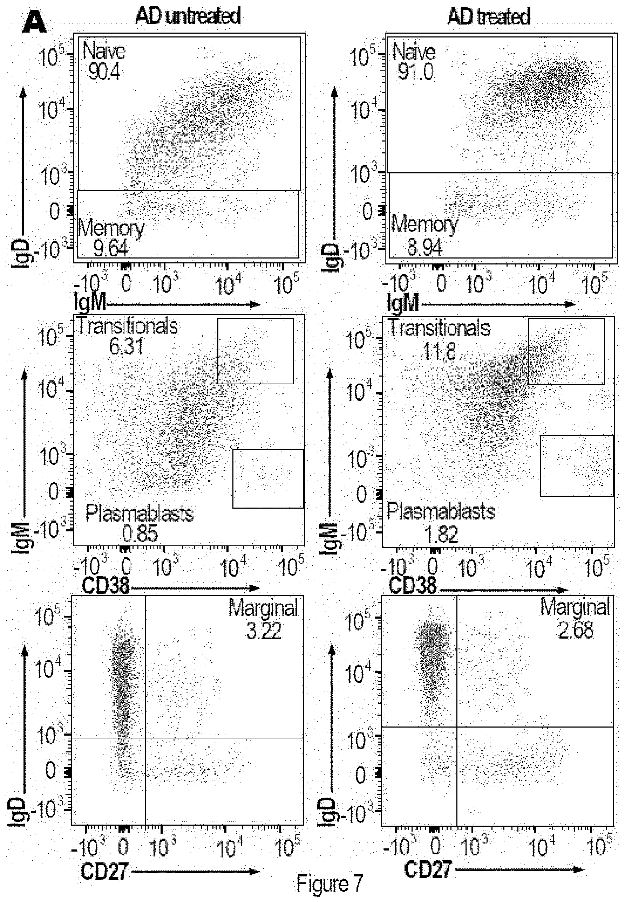

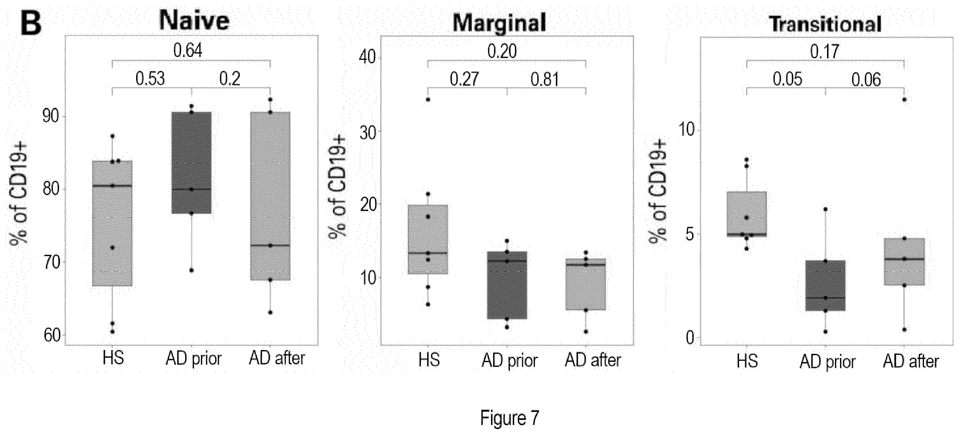

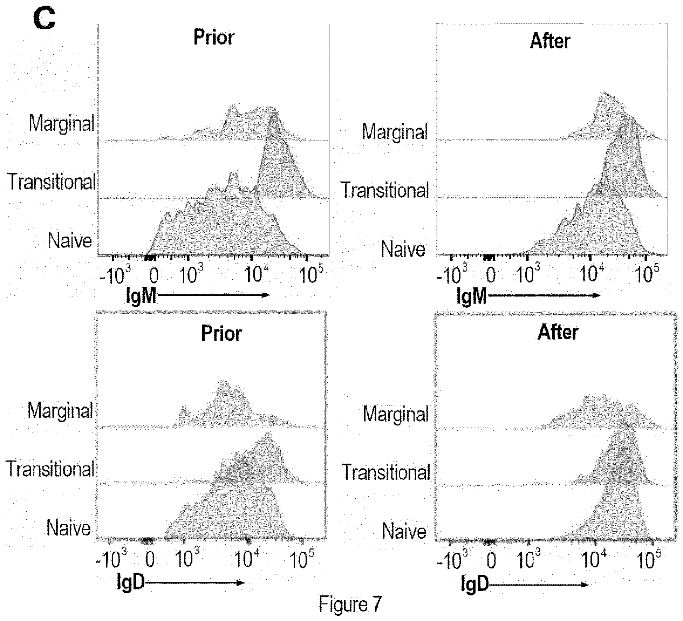

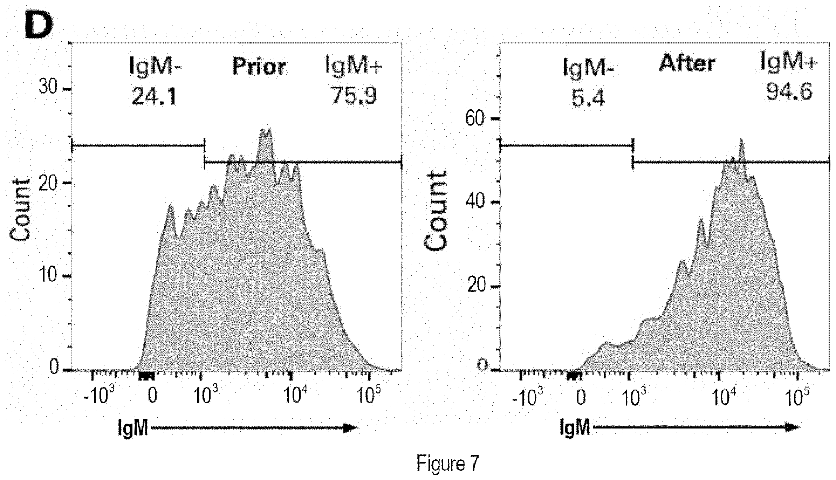

Figure 7 shows the results of flow cytometry (immunophenotyping) performed in peripheral blood from 5 patients with acutely decompensated cirrhosis included in the post-discovery Cohort 2 before and 24 hours after receiving albumin infusion. To distinguish the distinct B-cell subsets, a specific panel of B-cell markers was used including CD19, CD21, CD24, CD27, CD38, CD45, IgD and IgM (Panels A and B). B cells are defined as CD45+CD19+, and among B cells the different subpopulations are defined as: transitional B cells as IgM++CD38+++, naive B cells as IgD+IgM+, marginal B cells as IgD+CD27+, plasmablasts as IgM-CD38+++, and memory B cells as IgD-IgM-.Panel C represents the mean fluorescence intensity (MFI) of the IgM and IgD signal in naive, transitional and marginal B cells. Histograms representing the IgM levels in the surface of total B-cells are shown in Panel D.

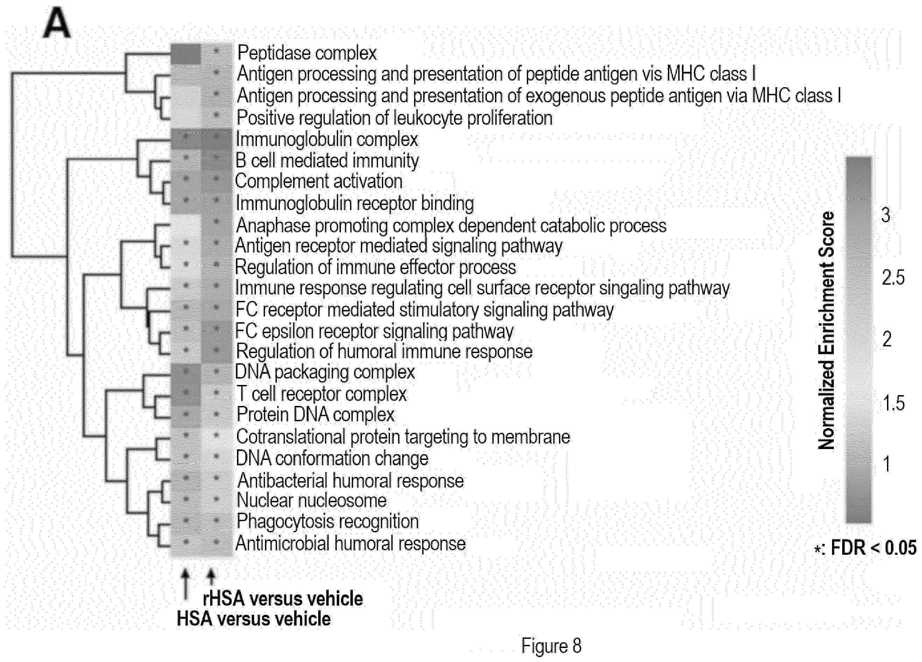

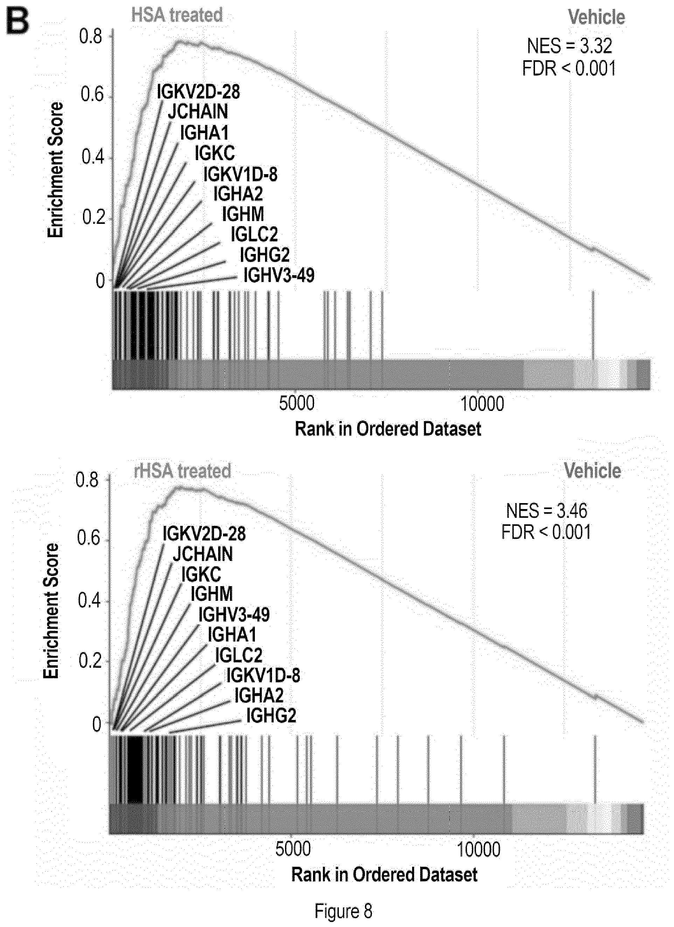

Figure 8 shows *ex vivo* effects of human serum albumin (human plasma-derived) and recombinant human serum albumin on transcriptional characteristics of PMBCs from 5 patients with acutely decompensated cirrhosis included in the post-discovery Cohort 3.

Panel A shows hierarchical cluster analysis of GSEA NES of 15 gene sets from PMBCs in 2 comparisons involving human serum albumin versus vehicle and recombinant human serum albumin versus vehicle. The 15 gene sets were identified from the analysis of the top 40 absolute values of significant NES in each comparison. Color gradient corresponds to the NES of gene sets that were downregulated (in blue) or upregulated (in red) in albumin treated relative to untreated samples. The star in certain cells of the heat map indicates significance (FOR <0.05). Panel B shows the GSEA enrichment plots of the immunoglobulin complex gene set in PMBCs isolated from acutely decompensated patients and incubated with human serum albumin (top of the panel) or recombinant human serum albumin (bottom of the panel) versus vehicle. The hash plot under GSEA curves shows where the members of the gene set appear in the ranked list of genes. The genes shown on the plots are representative of leading edge genes that are shared by the 2 comparisons. The NES and FDR testing the significance of each comparison are indicated.

Unless defined otherwise, all technical and scientific terms used herein have the same meaning as commonly understood by one of ordinary skill in the art pertinent to the methods and compositions described. As used herein, the following terms and phrases have the meanings ascribed to them unless specified otherwise.

The terms "a," "an," and "the" include plural referents, unless the context clearly indicates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Unless otherwise defined, all technical and scientific terms used herein have the same meaning as commonly understood by one of ordinary skill in the art to which this invention pertains. Exemplary methods and materials are described below, although methods and materials similar or equivalent to those described herein can also be used and will be apparent to those of skill in the art. All publications and other references mentioned herein are incorporated by reference in their entirety. In case of conflict, the present specification, including definitions, will control. The materials, methods, and examples are illustrative only and not intended to be limiting.

Each embodiment in this specification is to be applied mutatis mutandis to every other embodiment unless expressly stated otherwise.

The following terms, unless otherwise indicated, shall be understood to have the following meanings:
The term "treatment" or "treating" means any treatment of a disease or disorder in a subject, such as a mammal, including: preventing or protecting against the disease or disorder, that is, causing the clinical symptoms not to develop; inhibiting the disease or disorder, that is, arresting or suppressing the development of clinical symptoms; and/or relieving the disease or disorder that is, causing the regression of clinical symptoms. In some embodiments, the term treatment is used for the treatment of decompensated cirrhosis, acute liver failure, acute-on-chronic liver failure, severe sepsis and septic shock, Coronavirus Disease 19 (Covid19), severe polytraumatic conditions, severe burns, severe acute pancreatitis, hemophagocytic syndrome, heat-shock syndrome, acute ischemia-reperfusion syndrome, inflammation diseases, and cancer.

It will be understood by those skilled in the art that in human medicine, it is not always possible to distinguish between "preventing" and "suppressing" since the ultimate inductive event or events may be unknown, latent, or the patient is not ascertained until well after the occurrence of the event or events. Therefore, as used herein the term "prophylaxis" is intended as an element of "treatment" to encompass both "preventing" and "suppressing" as defined herein.

As used herein, the term "recombinant" refers to a biomolecule, e.g., a gene or protein, that (1) has been removed from its naturally occurring environment, (2) is not associated with all or a portion of a polynucleotide in which the gene is found in nature, (3) is operatively linked to a polynucleotide which it is not linked to in nature, or (4) does not occur in nature. The term "recombinant" can be used in reference to cloned DNA isolates, chemically synthesized polynucleotide analogs, or polynucleotide analogs that are biologically synthesized by heterologous systems, as well as proteins and/or mRNAs encoded by such nucleic acids. In some embodiments, the albumin is a recombinant albumin.

As used herein, the term "human plasma-derived" refers to a biomolecule, e.g., a gene or protein, which are obtained from a standard of pooled human plasma from donors. In some embodiments, the term human plasma-derived is used to refer a human plasma-derived albumin.

As used herein, the term "B-cell Receptors" refers to an immunoglobulin selected from the group consisting of an IgD subtype, an IgM subtype, and combinations thereof.

To address the potential effect of albumin on immunosuppression, the present inventors performed a prospective investigation using genomewide RNA-sequencing (RNA-seq) in two Cohorts of patients hospitalized with acutely decompensated cirrhosis derived from the PREDICT study. The discovery Cohort 1 included patients with Pre-ACLF who developed ACLF during hospitalization. The discovery Cohort 2 included patients with Pre-ACLF who developed septic shock during hospitalization. The present inventors also developed several *in vivo* and *ex vivo* studies to confirm the finding obtained in the discovery Cohorts. Both studies were performed on patients included in the PREDICT study, a prospective multicenter European observational investigation in 1241 patients hospitalized for 41 centers with acutely decompensated cirrhosis. The aim was to assess the clinical course during hospitalization and early follow-up (3-month). Clinical data and biobanking material were sequentially obtained for ancillary investigations. The discovery 1 and discovery 2 investigations were performed in 49 patients (37 and 12 patients, respectively) and 10 healthy subjects.

As used herein, the term "post-discovery Cohort 1" refers to PBMCs obtained from 9 patients with acutely decompensated cirrhosis treated *ex vivo* with either albumin or vehicle during 2 h.

As used herein, the term "post-discovery Cohort 2" refers to transcriptional characteristics in blood from 5 patients with acutely decompensated cirrhosis before and 24 hours after albumin treatment.

As used herein, the term "post-discovery Cohort 3" refers to PMBCs from 5 patients with acutely decompensated cirrhosis treated *ex vivo* with either human plasma derived albumin or recombinant human albumin during 24 h.

Hereinafter, the present invention is described in more detail with reference to illustrative examples, which do not constitute a limitation of the present invention.

### EXAMPLES

Example 1. Immunosuppression in diseases associated with systemic inflammation occurs in the context of a generalized impairment of the adaptive immune system. Albumin treatment improves the function of the adaptive immune system by activating B-cell function.

The discovery Cohort 1 included 37 patients with acutely decompensated cirrhosis (Table 1). Eighteen patients (albumin group) were selected because they (1) had pre-ACLF at enrollment and had not received albumin treatment during 10 days prior to enrolment (i.e., were in a pre-ACLF-untreated state at enrollment), (2) had developed ACLF within the month following enrolment and had received intravenous albumin within 10 days prior ACLF development (i.e. were in a ACLF-treated state at that time), (3) had no shock among the organ system failures that served to define ACLF, and (4) had had longitudinal blood collection for RNA-seq when they were in the pre-ACLF-untreated and ACLF-treated states (Fig. 1A). Using these criteria, the present inventors anticipated that comparison of longitudinally obtained RNA seq would capture changes in blood immune cells that were largely dependent on albumin administration.

The discovery Cohort 1 also included 19 patients (no albumin group) of the PREDICT study Cohort because they had ACLF, had not received intravenous albumin during the month prior to ACLF development (i.e., were in the ACLF-untreated state) and had blood collected for RNA-seq at ACLF development. Finally, the present inventors enrolled 10 age-matched healthy blood donor controls for whole -blood RNA-seq.

The median duration of albumin treatment was of 2.5 days (IQR, 2 to 6) and the median for albumin dose was of 40.0 g/day (IQR, 22.5 to 57.5). The median time elapsed between the pre-albumin and the post-albumin blood collections for RNA-seq was 11.5 days (IQR, 6.2 to 18.7), and the median time elapsed between the last albumin administration and subsequent blood collection for RNA-seq was 1.5 days (IQR, 0.0 to 2.7).

Progression from the pre-ACLF untreated state to the ACLF treated state was associated with significant increase in MELD and CLIF-C OF scores. However, except for serum creatinine, there were no significant changes regarding the rest of standard laboratory data and the plasma concentration of 14 inflammatory mediators. Similarly, except for serum creatinine, no between-group differences existed in patients at the ACLF-treated and ACLF-untreated states in clinical features, organ failures, prognostic scores, standard laboratory variables and inflammatory mediators. Therefore, the intensity of systemic inflammation was similar in all patients studied irrespective of the clinical or treatment state.

Effect of albumin on RNA-seq-inferred cell-population scores. Clinical blood count showed that patients, whichever their state, had neutrophilia and lymphopenia (Table 1). The present inventors used the RNA-seq-inferred cell scores to estimate cell subpopulations. The sum of RNA-seq-inferred lymphocyte scores correlated closely with the standard lymphocyte count in healthy subjects and patients confirming the accuracy of this estimation. Using this estimation, the present inventors found that RNA-seq-inferred population scores were increased for innate immune cells (neutrophils, basophils, monocytes, and dendritic cells) and decreased for adaptive immune cells (Th or CD4+, Tc or CD8+, B and NK cells) in patients in either of the 3 states relative to healthy subjects, with the exception of the RNA-seq-inferred population score of B-cells in the ACLF-treated state which did not significantly differ from that in healthy subjects (Fig. 2). Interestingly, RNA-seq-inferred B-cell population score showed a positive correlation with albumin dose (figure 3).

Together, these findings suggested that (1) systemic inflammation in acutely decompensated cirrhosis with and without ACLF is due to activation of the innate immune system and occurs in the setting of a significant under regulation of the adaptive immune system, which may be the mechanism of immunosuppression; (2) albumin selectively activates B-cell function without affecting the T-cell compartment.

Therefore, by doing this, albumin recues the B-cell from the generalized under-regulated dysfunction of the adaptive immune cells; (3) the effect of albumin on B-cells is dose-dependent.

| **Table 1. Characteristics of the Patients of the Albumin and No-Albumin Groups *** | | | | | |
|---|---|---|---|---|---|
| **Characteristic** | **Albumin Group (N=18)†** | | **No-albumin Group (N=19)†** | **P Value** | |
| | Pre-ACLF-Untreated | ACLF-Treated | ACLF-Untreated State | ACLF-Treated versus Pre-ACLF-Untreated | ACLF-Treated versus ACLF-Untreated |
| **Demographic feature** | | | | | |
| Age - yr | 60.9±8.9 | 60.9±8.9 | 59.1±10.8 | 1 | 0.58 |
| Male sex - n (%) | 13 (72) | 13(72) | 12 (63) | 1 | 0.81 |

| **Markers of organ function** | | | | | |
|---|---|---|---|---|---|
| MELD score‡ | 18.8±5.5 | 26.6±5.3 | 24.0±7.5 | <0.001 | 0.24 |
| CLIF-C OF score§ | 6.8±1.0 | 9.1±1.7 | 8.9±1.5 | <0.001 | 0.76 |

| **Organ system failure - n (%)** | | | | | |
|---|---|---|---|---|---|
| Liver failure | 4 (22) | 4 (22) | 6 (32) | 1 | 0.79 |
| Kidney failure | 0(0) | 15 (83) | 13(68) | <0.001 | 0.50 |
| Cerebral failure | 0(0) | 3 (17) | 1 (5) | 0.23 | 0.56 |
| Coagulation failure | 0 (0) | 2(11) | 4(22) | 0.47 | 0.65 |
| Respiratory failure | 0(0) | 2(11) | 0(0) | 0.47 | 0.44 |

| **ACLF grade - n (%)**** | | | | | |
|---|---|---|---|---|---|
| Grade 1 | - | 12 (67) | 13 (72) | - | 1 |
| Grade 2-3 | - | 6 (33) | 5 (28) | - | 1 |

| **Precipitating events** | | | | | |
|---|---|---|---|---|---|
| Infection as pre-ACLF or ACLF precipitant - n (%) | 7 (39) | 8 (44) | 7 (37) | 1 | 0.89 |
| Alcoholic hepatitis as pre-ACLF or ACLF precipitant - n (%) | 7 (44) | 6 (33) | 7 (44) | 0.79 | 1 |

| **Laboratory data** | | | | | |
|---|---|---|---|---|---|
| INR-median (IQR) | 1.5 (1.3-1.6) | 1.6 (1.5-1.8) | 1.4 (1.3-2.2) | 0.12 | 0.40 |
| Median total bilirubin (IQR) - mg/l | 3.3 (1.8-5.5) | 3.2 (1.8-6.2) | 5.0 (1.0-13.5) | 0.75 | 0.78 |
| Median serum creatinine (IQR)-mg/dl | 1.3 (1.1-1.6) | 2.7 (2.1-3.3) | 2.1 (1.4-2.4) | <0.001 | 0.01 |
| Serum sodium-mmol/l | 132.3±5.0 | 134.2±7.6 | 132.6±6.9 | 0.38 | 0.49 |
| Median serum albumin (IQR) - g/dl | 2.9 (2.4-3.3) | 3.2 (2.6-4.3) | 3.0 (2.8-3.1) | 0.22 | 0.52 |
| Median white-cell count (IQR) - cells/mm³ | 8.3 (7.1-9.9) | 8.3 (5.5-13.2) | 8.1 (5.8-12.4) | 0.69 | 0.99 |
| Median absolute lymphocyte count (IQR) - ×10³/mm³ | 1.1 (0.7-1.6) | 1.1 (0.8-1.6) | 1.2 (0.8-1.8) | 0.86 | 0.55 |
| Median absolute monocyte count (IQR) - ×10³/mm³ | 0.7 (0.6-1.0) | 1.1 (0.6-1.2) | 0.7 (0.6-0.9) | 0.58 | 0.43 |
| Median absolute neutrophil count (IQR) - ×10³/mm³ | 6.1 (4.2-6.9) | 6.4 (3.6-10.1) | 4.5 (3.0-7.8) | 0.63 | 0.36 |

| **Median blood levels of protein mediators of inflammation (IQR) - pg/ml** | | | | | |
|---|---|---|---|---|---|
| Eotaxin | 71.0 (46.7-113.1) | 78.7 (58.6-104.1) | 64.3 (50.1-121.9) | 0.35 | 0.72 |
| G-CSF | 13.5 (3.6-35.8) | 21.7 (12.0-75.1) | 16.9 (3.6-70.9) | 0.51 | 0.73 |
| Interferon-α2 | 17.0 (2.8-28.8) | 19.0 (8.9-24.6) | 17.6 (8.2-22.7) | 0.74 | 0.81 |
| Interferon-γ | 24.9 (5.7-125.9) | 16.4 (5.1-60.3) | 25.9 (7.9-45.8) | 0.51 | 0.71 |
| Interleukin-1α | 4.3 (2.8-7.1) | 1.4(0.8-3.7) | 2.0 (0.3-3.9) | 0.11 | 0.74 |
| Interleukin-1β | 5.7 (3.5 -13.5) | 4.6 (2.3-10.4) | 5.2 (1.9-8.7) | 0.48 | 0.84 |
| Interleukin-6 | 14.8 (8.8-38.1) | 26.2 (10.5-68.4) | 16.9 (8.8-23.1) | 0.57 | 0.29 |
| Interleukin-8 | 8.6 (4.8-14.7) | 13.0 (3.8-15.3) | 4.3 (1.0-9.0) | 0.91 | 0.11 |
| Interleukin-10 | 10.0 (3.3-30.4) | 5.8 (3.3-24.3) | 4.7 (3.2-5.8) | 0.83 | 0.56 |
| IP-10 (CXCL10) | 185.6 (123.1-375.5) | 207.3 (168.6-400) | 236.6 (140.6-486.7) | 0.55 | 0.75 |
| MIP-1α (CCL3) | 11.4 (9.0-22.6) | 13.8 (6.8-23.8) | 13.3 (4.5-19.4) | 1 | 0.66 |
| MIP-1β (CCL4) | 15.6 (12.5-17.6) | 17.2 (12.8-22.8) | 15.2 (11.7-19.6) | 0.42 | 0.47 |
| Tumor necrosis factor α | 31.9 (21.5-41.1) | 27.7 (20.4-49.7) | 35.6 (20.8-49.3) | 0.89 | 0.79 |
| VEGF | 3.6 (0.8-6.2) | 3.8 (2.8-9.8) | 7.6 (4.7-11.7) | 0.51 | 0.27 |
| * Plus-minus values are means ±SD. To convert the values for serum creatinine to micromoles per liter, multiply by 88.4. To convert the values for bilirubin to micromoles per liter, multiply by 17.1. Pre-ACLF denotes pre-acute-on-chronic liver failure, ACLF acute-on-chronic liver failure, MELD Model for End-Stage Liver Disease, CLIF-C Chronic Liver Failure Consortium, OF organ failure, INR Intemational Normalized Ratio, IQR interquartile range, G-CSF granulocyte-colony stimulating factor, IP-10 interferon-inducible protein 10, CXCL C-X-C motif chemokine ligand, MCP-1 monocyte chemoattractant protein 1, CCL CC motif chemokine ligand, MIP macrophage inflammatory protein, and VEGF vascular endothelial growth factor. | | | | | |
| † Eighteen patients with acutely decompensated cirrhosis were longitudinally studied before anc after they had received intravenous albumin. These 18 patients had pre-ACLF before albumin and ACLF after albumin treatment (indicating that they were first in a pre-ACLF-untreated state anc then in ACLF-treated state). 19 patients with ACLF who had not received albumin (and therefore were investigated when they were in the untreated state). | | | | | |
| ‡ The MELD score is calculated (www.mayoclinic.org/meld/mayomodel7.html) as follows: (9.57×log creatinine in milligrams per deciliter)+(3.78×log bilirubin in milligrams per deciliter)+(11.20×log international normalized ratio)+6.43. Scores range from 6 to 42, with higher scores indicating a worse prognosis. | | | | | |
| § The CLIF-C OF score includes subscores ranging from 1 (close to normal) to 3 (most abnormal) for each of the six major organ systems (kidneys, lungs, liver, coagulation, brain, circulation). Aggregated scores range from 6 to 18, with higher scores indicating more severe organ failure. ¶ According to the CLIF-C OF scale, liver failure is defined by serum bilirubin levels ≥12 mg/dl, kidney failure by serum creatinine ≥2 mg/dl or use of renal-replacement therapy, cerebral failure by hepatic encephalopathy Grade 3-4 in the West Haven classification or use of mechanical ventilation because of HE, circulation failure by the use of va so pressors including terlipressin, anc respiratory failure by PaO2/FiO2 ≤200 or SpO2/FiO2 ≤214, or use of mechanical ventilation for other reason than hepatic encephalopathy. PaO2 denotes partial pressure of arterial oxygen, FiO2 fraction of inspired oxygen, and SpO2 pulse oximetric saturation. | | | | | |
| ** According the CLIF-C definition, the grade of ACLF is defined by organ system failures identified according to the CLIF-C OF scale. ACLF grade 1 is defined by the presence of (1) single kidney failure, or (2) single liver, coagulation, circulatory or lung failure associated with creatinine levels ranging from 1.5 to 1.9 mg/dl or hepatic encephalopathy grade 1 or 2, or both; ACLF grade 2 by the presence of 2 organ system failures; and ACLF grade 3 by the presence of 3 organ failures or more. | | | | | |

Albumin activates many gene sets related with immunoglobulins synthesis and processes. The present inventors used gene-set enrichment analysis using whole-blood RNA-seq data to identify gene sets and processes that showed upregulation or downregulation when patients were in either of the 3 states (pre-ACLF-untreated, ACLF treated and ACLF-untreated) as compared with healthy subjects. The number of significantly enriched (upregulated or downregulated) gene sets in the pre-ACLF-untreated, ACLF-treated and ACLF-untreated states with respect to healthy subjects were 109, 62 and 134, indicating a significant effect of albumin in whole-blood gene expression. The analysis of the top 40 absolute values of significant normalized enrichment scores in each comparison showed a similarity of several significantly enriched set of genes across the three comparisons (Fig. 4A). For example, gene sets related to neutrophil granules were significantly overexpressed in patients relative to healthy subjects. In contrast, gene sets related to T-cells (for example, T-cell receptor complex) were significantly underexpressed in patients relative to healthy subjects. Importantly, 3 gene sets related to B-cells (immunoglobulin complex, phagocytosis recognition, and immunoglobulin complex circulating) with decreased expression in the untreated states, normalized their expression in the ACLF-treated state (Figs. 4A and 4B), indicating that albumin "rescued" immunoglobulin gene expression in B-cells.

To further evaluate the effects of albumin treatment the present inventors conducted 2 additional gene- set enrichment analysis comparing data in the ACLF-treated state versus those in the pre-ACLF-untreated and in the ACLF-untreated states. With a false discovery rate of less than 5 %, the present inventors identified 98 and 78 gene sets, respectively, showing significant enrichment, of which almost all were upregulated in treated patients versus untreated patients. The analysis of the top 40 absolute values of significant normalized enrichment scores in each comparison showed a similarity of several significantly enriched gene sets in the two comparisons. In particular, 10 gene sets (i.e. immunoglobulin complex, humoral immune response mediated by circulating immunoglobulin, phagocytosis recognition, regulation of humoral immune response, cell recognition, B-cell receptor signaling pathway, positive regulation of B-cell activation, Fc receptor signaling pathway, phagocytosis and immunoglobulin production) were significantly upregulated in treated patients relative to untreated patients (Figs. 4C and 4D). Of note, in each comparison, immunoglobulins were prominent among - leading-edge genes that contributes to enrichment of each of the ten gene set.

Moreover, focusing on the immunoglobulin complex gene set, the present inventors found that leading-edge genes overlapped in the two comparisons (Figs. 4D and 4E). A detailed list of immunoglobulin genes, including variable, diversity, joining and constant genes, which contribute to the immunogloigulin chain synthesis, that are upregulated by albumin is shown in table 2. Combination of these genes provides an immunoglobulin gene score (IGsc) informing about the response of B cells to albumin.

**Table 2. List of immunoglobulin genes composing the gene score of the response of B cells from patients with AD cirrhosis to albumin**

| |
|---|
| IGKV2D.24 |
| IGHG3 |
| IGKV2D.30 |
| IGKJ1 |
| IGLV3.12 |
| IGKV3D.11 |
| IGKV1D.33 |
| IGHV2.5 |
| IGHV1.69.2 |
| IGLC7 |
| IGKV6.21 |
| IGKV2D.29 |
| IGKV1D.17 |
| IGLV5.37 |
| IGLV10.54 |
| IGKV6D.21 |
| IGKV1D.13 |
| IGKV2.28 |
| IGHV3.66 |
| IGHE |
| IGHV3.64 |
| SYK |
| IGHV2.26 |
| IGKV2D.28 |
| IGHV4.28 |
| CD79A |
| IGLV5.45 |
| IGHV1.69 |
| IGHV4.4 |
| IGKV1.39 |
| IGKC |
| IGKV4.1 |
| IGLV2.14 |
| IGHG2 |
| IGHM |
| IGHV6.1 |
| IGHV3.7 |
| IGHV3.13 |
| IGHV3.23 |
| IGHV4.34 |
| IGHV3.48 |
| IGHV3.49 |
| IGLV9.49 |
| IGHV3.74 |
| IGKV1D.8 |

Together these findings confirmed the robustness of the enrichment analysis in detecting a widespread increase in the expression of immunoglobulin genes in B-cells from patients who had received albumin.

Example 2. Immunosuppression in septic shock occurs in the context of a generalized impairment of the adaptive immune system. Albumin treatment improves the function of the adaptive immune system by activating B-cell function.

The discovery Cohort 2 consisted in 12 patients selected from the PREDICT study who had (1) pre-ACLF at enrolment, (2) had not received intravenous albumin during the 10 days prior to enrolment, (3) had developed septic shock (a form of ACLF) during hospitalization and received intravenous albumin for circulatory support during the course of septic shock, from the pre-ACLF state up to the development of ACLF, and (4) had had longitudinal blood collection for whole-blood RNA-seq prior to and after albumin. Clinical characteristics of the patients are indicated in table 3. Using gene set enrichment analysis, data obtained in these patients prior to and after albumin treatment were compared to those obtained in 10 healthy subjects (Fig. 5A). In each comparison, gene sets related to neutrophil granules were significantly overexpressed, while gene sets related to T and B-cells were significantly underexpressed in patients relative to healthy subjects. This pattern of gene set enrichment has also been reported in patients without cirrhosis with septic shock. The most relevant observation of the discovery study 2 was that gene sets involving immunoglobulin genes (i.e., the immunoglobulin complex), which were significantly underexpressed in patients with septic shock relative to healthy subjects before treatment, normalized their expression after albumin administration (Fig. 5A and 5B).

Together, the present findings showed that intravenous albumin "rescued" immunoglobulin gen set expression in patients with cirrhosis and septic shock. The pattern of gene-set enrichment observed in our patients with cirrhosis and septic shock is like that observed by other investigators in patients without cirrhosis and septic shock, suggesting the results could be extended to any patient septic shock, independently of the primary disease.

| **Table 3. Characteristics of the Patients with Septic Shock treated with albumin** | | | |
|---|---|---|---|
| | **Longitudinal data** | | **P Value** |
| **Characteristic** | Pre-ACLF-Untreated (N=12) | ACLF-Septic Shock-Treated (N=12) | ACLF-Septic Shock-Treated versus Pre-ACLF-Untreated |
| **Demographic feature** | | | |
| Age-yr | 61.1±6.5 | 61.1±6.5 | 1 |
| Male sex - n (%) | 8 (67) | 8 (67) | 1 |

| **Markers of organ function** | | | |
|---|---|---|---|
| MELD secre‡ | 19.7±5 | 27.6±8.5 | 0.01 |
| CLIF-C OF score§ | 7.2±0.9 | 12.82.1 | <0.001 |

| **Organ system failure - n (%)** | | | |
|---|---|---|---|
| Liver failure | 2 (17) | 3 (25) | 1 |
| Kidney failure | 0 (0) | 8 (67) | <0.001 |
| Cerebral failure | 0 (0) | 3 (25) | 0.22 |
| Coagulation failure | 0 (0) | 2 (17) | 0.48 |
| Circulatory failure | 0 (0) | 12 (100) | <0.001 |
| Respiratory failure | 0 (0) | 7 (58) | <0.001 |

| **ACLF grade - n (%)**** | | | |
|---|---|---|---|
| Grade 1 | 0 (0) | 1 (8) | 1 |
| Grade 2-3 | 0 (0) | 11 (92) | <0.001 |

| **Precipitating events** | | | |
|---|---|---|---|
| Infection as pre-ACLF or ACLF precipitant- n (%) | 7 (58) | 12 (100) | 0.01 |
| Alcoholic hepatitis as pre-ACLF or ACLF precipitant - n (%) | 7 (58) | 8 (67) | 1 |

| **Laboratory data** | | | |
|---|---|---|---|
| INR-median (IQR) | 1.7 (1.6-1.8) | 1.8 (1.6-2.1) | 0.31 |
| Median total bilirubin (IQR) - mg/dl | 5.5 (3.2-10.2) | 4.7 (3-12.1) | 0.98 |
| Median serum creatinine (IQR) - mg/dl | 1.1 (0.8-1.4) | 2.8 (1.1-3.5) | <0.001 |
| Serum sodium - mmol/l | 129.9±6.8 | 131.8±9.6 | 0.49 |
| Median serum albumin (IQR) - g/dl | 2.3 (2.1-2.8) | 2.6 (2.2-3.1) | 0.81 |
| Median white-cell count (IQR) - cells/mm³ | 8.5 (6.6-11.1) | 12.5 (10.4-16) | 0.03 |
| Median absolute neutrophil count (IQR) - cells/mm³ | 6.6 (4.7-9.3) | 11.2 (8.8-13) | 0.11 |
| Median absolute lymphocyte count (IQR)-cells/mm³ | 0.8 (0.5-0.9) | 1.2 (0.4-1.6) | 0.74 |
| Median absolute monocyte count (IQR) - cells/mm³ | 0.8 (0.4-1.1) | 1 (0.9-1.4) | 0.27 |
| Eotaxin | 61.5 (39.7-72.5) | 95.8 (58.9-121.4) | 0.13 |
| G-CSF | 21.6 (11.5-388.1) | 24.5 (6.1-70) | 0.81 |
| Interferon-α2 | 8.2 (1.9-15.6) | 12.5 (1.6-38.7) | 0.29 |
| Interferon-γ | 47.3 (16-68.4) | 39 (22.9-109.8) | 0.19 |
| Interleukin-1α | 1.7 (0.9-9.3) | 3.8 (1.7-9.2) | 0.58 |
| Interleukin-1β | 5.4 (2.4-9.9) | 7.5 (2.3-17) | 0.38 |
| Interleukin-1ra | 4.7 (2.2-33) | 16.9 (7.9-22.8) | 0.79 |
| Interleukin-6 | 41 (19.8-119.9) | 156.7 (53-286.4) | 0.47 |
| Interleukin-8 | 4.2 (2.4-7) | 11.8 (6-19.4) | 0.34 |
| Interleukin-10 | 13(4-41.8) | 16.4(7.9-26.4) | 0.95 |
| IP-10 (CXCL10) | 248 (159.6-371.5) | 378.4 (199.4-895.3) | 0.42 |
| MCP-1 (CCL2) | 159.8 (102.6-366.3) | 526.2 (362.8-669.7) | <0.001 |
| MIP-1α (CCL3) | 12.9 (5.1-21.9) | 22.8 (11.8-28.9) | 0.28 |
| MIP-1β (CCL4) | 16 (10.3-27.4) | 28.8 (16.3-50.4) | 0.27 |
| Tumor necrosis factor α | 25.7 (17-46.1) | 52.9 (28.4-70.4) | 0.11 |
| VEGF | 6 (0.9-8.5) | 11.1 (2.4-17.9) | 0.02 |
| * Plus-minus values are means ±SD. To convert the values for serum creatinine to micromoles per liter, multiply by 88.4. To convert the values for bilirubin to micromoles per liter, multiply by 17.1. Pre-ACLF denotes pre-acute-on-chronic liver failure, ACLF acute-on-chronic liver failure, MELD Model for End-Stage Liver Disease, CLIF-C Chronic Liver Failure Consortium, OF organ failure, INR International Normalized Ratio, IQR interquartile range, G-CSF granulocyte-colony stimulating factor, IP-10 interferon-inducible protein 10, CXCL C-X-C motif chemokine ligand, MCP-1 monocyte chemoattractant protein 1, CCL CC motif chemokine ligand, MIP macrophage inflammatory protein, and VEGF vascular endothelial growth factor.† Twelve patients with acutely decompensated cirrhosis were longitudinally studied before and after they had received intravenous albumin. These 12 patients with pre-ACLF before albumin treatment developed sepsis-related ACLF after albumin treatment (indicating that they were first in a pre-ACLF-untreated state and then in ACLF-Septic Shock-treated state). | | | |
| ‡ The MELD score is calculated (www.mayoclinic.org/meld/mayomodel7.html) as follows: (9.57×log creatinine in milligrams per deciliter)+(3.78×log bilirubin in milligrams per deciliter)+(11.20×log international normalized ratio)+6.43. Scores range from 6 to 42, with higher scores indicating a worse prognosis. | | | |
| § The CLIF-C OF score includes subscores ranging from 1 (close to normal) to 3 (most abnormal) for each of the six major organ systems (kidneys, lungs, liver, coagulation, brain, circulation). Aggregated scores range from 6 to 18, with higher scores indicating more severe organ failure. | | | |
| ¶ According to the CLIF-C OF scale, liver failure is defined by serum bilirubin levels ≥12 mg/dl, kidney failure by serum creatinine ≥2 mg/dl or use of renal-replacement therapy, cerebral failure by hepatic encephalopathy Grade 3-4 in the West Haven classification or use of mechanical ventilation because of HE, circulation failure by the use of vasopressors including terlipressin, and respiratory failure by PaO₂/FiO₂ ≤200 or SpO₂/FiO₂≤214, or use of mechanical ventilation for other reason than hepatic encephalopathy. PaO₂ denotes partial pressure of arterial oxygen, FiO₂ fraction of inspired oxygen, and SpO₂ pulse oximetric saturation. | | | |
| ** According the CLIF-C definition, the grade of ACLF is defined by organ system failures identified according to the CLIF-C OF scale. ACLF grade 1 is defined by the presence of (1) single kidney failure, or (2) single liver, coagulation, circulatory or lung failure associated with creatinine levels ranging from 1.5 to 1.9 mg/dl or hepatic encephalopathy grade 1 or 2, or both; ACLF grade 2 by the presence of 2 organ system failures; and ACLF grade 3 by the presence of 3 organ failures or more. | | | |

Example 3, together with examples 4 and 5 consisted in three studies designed to confirm the findings obtained in the Discovery cohorts 1 and 2.

Example 3. Single-cell RNA-sequencing (ScRNA Seq) of peripheral blood mononuclear cells (PBMCs).

Example 3 study was performed to explore the early effects of albumin on cell state plasticity in the B-cell and monocyte compartment. Fresh PMBCs from 9 additional patients included in the post-discovery Cohort 1 with acutely decompensated cirrhosis from the PREDICT study were exposed ex *vivo* to human albumin or vehicle, and after 2 hours of exposure PMBC ScRNA-seq was performed. Analyzing 1,834 B-cells merged from the 2 experimental conditions (albumin or vehicle) the present inventors identified three main B-cell population, naive (unexposed to an antigen), memory, and plasma cells (Fig. 6A). In the naive compartment, the present inventors identified four transcriptionally distinct subpopulations, among them two transitional (T1 and T2) cell states (Fig. 6A).

After exposure to human albumin, the present inventors observed specific changes in the transcriptional profile in the naive compartment with a significant increase in the abundance of transitional B subtypes (T1 and T2) (p-value < 0.05) (Figs. 6B and 6C). Transitional cells increase is based on naive decrease (see Fig. 6B). Gene Ontology gene sets related to regulation of immune response, regulation of immune effector process and phagocytosis were significantly enriched among genes that were markers of transitional B-cells (Fig. 6D), finding consistent with functions of transitional B-cells.

Signals produced by myeloid cells may participate in B-cell activation. Therefore, the present inventors analyzed 20,396 myeloid cells merged from the scRNA-seq data obtained in patients PMBCs under the 2 experimental conditions. Five clusters were related to monocytes, including classical monocytes, nonclassical monocytes, inflammatory intermediates monocytes, intermediate activated monocytes, and regulatory intermediate inflammatory monocytes (Fig. 6E). During exposure to human albumin, the present inventors observed a significant increase in the abundance of nonclassical and regulatory intermediate monocytes (p-value < 0.05), and a significant decrease in the abundance of activated intermediate monocytes (p-value <0.05) (Figs. 6F and 6G). Together these findings showed that *ex vivo* exposure of PMBCs to human albumin resulted in transcriptional changes in transitional B-cells and monocytes, in particular regulatory intermediate monocytes. Strikingly, B-cell-related gene sets were significantly enriched among gene markers of regulatory intermediate monocytes (figure 6H) and vice versa (figure 6D), suggesting that the increase abundance of transitional B-cells induced by albumin may be mediated, at least in part, by effects on regulatory intermediate monocytes.

Together these findings showed that (1) a major *ex vivo* effect of human albumin was to increase the gene inferred abundance of transitional B-cells among the blood cell compartment within 2 hours after the onset of the incubation period; (2) This extremely rapid response to albumin, the dose gene response relationship observed in the discovery study 1, and the increased gene inferred abundance of transitional B-cells and regulatory intermediate monocytes in response to albumin found in this post-discovery study, suggest that albumin activates B-cell function in a T-independent manner through a process mediated by regulatory monocytes.

Example 4. B-cell immunophenotyping.

The study was performed to explore the early response in patients receiving a single-day treatment with albumin at immunoglobulin protein level, by means of the intensity of the IgM and IgD signal on the surface of B-cells (naïve, marginal, and transitional cells). The study was performed in an additional group of 5 patients hospitalized with acutely decompensated cirrhosis who did not received albumin within 10 days before enrolment. Blood samples were obtained for PBMC immunophenotyping by just before and 24 h after albumin treatment. Among the distinct B-cells, transitional B-cells defined by coexpression of IgM and CD38 were significantly increased in patients treated with albumin (Figs. 7A and 7B). Similar frequencies of naïve (defined as IgD+IgM+) and marginal (defined as IgD+CD27+) B-cells (Fig. 7A and 7B) as well as memory (defined as IgD-IgM-) B-cells were observed before and after treatment (Fig. 7A). Results showed increases in IgD and IgM protein expression at the surface of naive, marginal and transitional B-cells (Fig. 7C). The albumin-induced upregulation of IgM levels is better appreciated by computing the total IgM signal on the surface of B-cells (Fig. 7D).

These data indicate that albumin induction of immunoglobulin genes results in rapid expression in immunoglobulin protein at cell surface, increasing thus B-cell Receptor density in a B-cell population, supporting a T-independent activation of B-cell.

Example 5. *Ex vivo* effects of human serum albumin and recombinant human albumin in PMBCs from patients hospitalized with acutely decompensated cirrhosis.

To investigate if B-cell response to human serum albumin was due to albumin or to other protein contained in the albumin solution (>95 % pure), the present inventors compared the *ex vivo* effects of human plasma-derived (Albutein^{®}) or recombinant albumin versus vehicle on RNA-seq in PMBCs from 5 patients with acutely decompensated cirrhosis. Hierarchical cluster analysis of top 40 absolute values of significant normalized enrichment score identified 4 overexpressed gene-sets related with immunoglobulins in both comparisons, including immunoglobulin complex, B-cell mediated immunity, complement activation, and immunoglobulin receptor binding (Fig 8A). Focusing on the immunoglobulin complex genes, the present inventors found that leading-edge genes overlapped in the two comparisons involving cells treated with human serum albumin or recombinant albumin (Fig. 8B).

Since albumin was presumably the only molecule shared by both the human serum and recombinant albumin solutions, these data support that B-cell responses in the present invention were related to the albumin molecule and not to other contaminating proteins.

## Claims

1. Composition comprising human albumin for regulating B-cell function in a patient suffering from systemic inflammatory response syndrome,
wherein the albumin is administered to the patient in a dose sufficient to increase B-cell Receptor density in a B-cell population selected from the group consisting of naïve B-cells, transitional B-cells, marginal B-cells and combinations thereof
such that the B-cell Receptor density increases by at least about 10 % compared to the pre-treatment B-cell Receptor density, as determined by flow cytometry.

2. The composition of claim 1, wherein the patient suffering from systemic inflammatory response syndrome is a sepsis patient or a patient with a liver condition.

3. The composition of claim 1, wherein the patient suffering from systemic inflammatory response syndrome has a liver condition selected from the group consisting of decompensated cirrhosis, pre-acute-on-chronic liver failure, and acute-on-chronic liver failure.

4. The composition of any preceding claim, wherein the B-cell Receptors comprise an immunoglobulin selected from the group consisting of an IgD subtype, an IgM subtype, and combinations thereof.

5. The composition of any preceding claim, wherein the B-cell Receptor density increases by at least about 20 % compared to the pre-treatment B-cell Receptor density.

6. Composition comprising human albumin for the treatment of defective B-cell function in a patient in need thereof.

7. The composition according to claim 6, wherein said defective B-cell function is related to a disease selected from the group comprising systemic inflammatory response syndrome, decompensated cirrhosis, acute liver failure, acute on chronic liver failure, severe sepsis and septic shock, Coronavirus 19 Disease (Covid 19), severe polytraumatic conditions, severe burns, severe acute pancreatitis, hemophagocytic syndrome, heat-shock syndrome, acute ischemia-reperfusion syndrome, inflammation diseases, and cancer.

8. The composition according to claim 7, wherein said cancer is selected from the group comprising Hodgkin lymphoma, breast cancer, cervical cancer, bladder cancer, colon cancer, head and neck cancer, liver cancer, lung cancer, renal cell cancer, skin cancer, stomach cancer and rectal cancer.

9. The composition of anyone of claims 1 to 5, or 6 to 8 wherein the albumin is administered by an administration route selected from the group consisting of intravenous, subcutaneous, intramuscular, intradermal, intraperitoneal, intrapulmonary, intranasal, oral, rectal, and combinations thereof.

10. The composition of any preceding claim wherein the albumin is administered at multiple intervals as part of a multiple-dosage regimen.

11. The composition of claim 10 wherein the multiple dosing regimen comprises multiple portion doses administered at equally spaced intervals of from about 1 day to about 30 days; or
wherein the multiple dosing regimen comprises multiple portion doses administered at unequally spaced intervals of from about 1 day to about 30 days; or
wherein the multiple dosing regimen comprises two or more administrations up to a total cumulative dose.

12. The composition of claims 10 to 11 wherein the albumin is administered at individual doses of about 5 g/interval to about 500 g/interval as part of a multiple-dosage regimen; or
wherein the albumin is administered at individual doses of about 20 g/interval to about 200 g/interval as part of a multiple-dosage regimen.

13. The composition of claims 10 to 12 wherein the dosing interval is every 10 days, or less.

14. The composition of any preceding claim, wherein the albumin is human plasma-derived albumin or recombinant albumin.

15. The composition of any preceding claim, wherein the concentration of albumin is between 4 % and 25 % (w/v); or
wherein the concentration of albumin is 20 % (w/v).
